# EUROPEAN PATENT SPECIFICATION

(11) **EP 4 415 610 B1**
(45) Date of publication and mention of the grant of the patent: **12.11.2025**
(21) Application number: 22792923.9
(22) Date of filing: 03.10.2022
(51) Int. Cl.: A61B 5/0205, A61B 5/024, A61B 5/0245, A61B 5/00

(54) **MEDICAL WATCH FOR MEASURING PHYSIOLOGICAL PARAMETERS**
MEDIZINISCHE UHR ZUR MESSUNG PHYSIOLOGISCHER PARAMETER
MONTRE MÉDICALE POUR MESURE DES PARAMÈTRES PHYSIOLOGIQUES

(30) Priority: 12.10.2021 IL 28721621
(43) Date of publication of application: 21.08.2024
(73) Proprietor: Cardiacsense Ltd., 3079870 Caesarea (IL)
(72) Inventor: OFEK, Guy, 1790600 Shimshit (IL); WAINSTEIN, Andres, 5258806 Ramat Gan (IL); KOUPERMAN, Igor, 2060000 Yokneam (IL); SPEKTOR, Boris, 3464122 Haifa (IL); SHEMESH, Eldad, 3057203 Binyamina (IL)
(74) Representative: Benatov, Samuil Gabriel
(86) International application number: PCT/IL2022/051054
(87) International publication number: WO 2023/062623

(56) References cited:
- CN-A- 106 419 880
- US-A1- 2016 183 818
- US-A1- 2017 020 399
- US-A1- 2018 035 943
- US-A1- 2019 059 756
- US-A1- 2021 193 977
- US-B1- 10 485 477

## Description

### TECHNOLOGICAL FIELD

The present disclosure is in the field of medical devices, in particular wearable devices for medical use.

### BACKGROUND ART

References considered to be relevant as background to the presently disclosed subject matter are listed below:
- WO 2019/215723
- CN211560078
- US2020375493
- WO2021057873

Acknowledgement of the above references herein is not to be inferred as meaning that these are in any way relevant to the patentability of the presently disclosed subject matter. Document CN106419880 is background art enabling continuous ECG monitoring using a medical watch.

### GENERAL DESCRIPTION

The present disclosure provides, in a first aspect, a medical watch for allowing various measurements of physiological parameters of the subject wearing it. The medical watch is designed to facilitate a simultaneous measurement of at least two types of measurements that require placing a finger on a specific spot in order to perform the measurement. For allowing the simultaneous measurement, the watch is designed with a peripheral rim or a peripheral section (used herein interchangeably throughout the application with the term "peripheral member") that accommodates two sensing surfaces of different sensors that are located at substantially opposite sides of the peripheral rim to allow easily placing of the thumb and an additional finger simultaneously on the two sensing surfaces. For example, the two sensing surfaces can be of a pulse oximetry sensor and an ECG electrode. In this example, the medical watch is capable of performing simultaneous measurement of SpO₂ data and ECG data of the subject and correlate them. In some embodiments, the medical watch can include additional sensors, such as a PPG sensor, to collect additional physiological data from the subject and correlate between all the acquired medical data at a single time point.

As can be appreciated throughout the application, there are references to elements or surfaces that are formed on the peripheral member, both representing parts of sensor that are intended for performing measurements of physiological parameters thereby.

It is to be noted that any combination of the described embodiments with respect to any aspect of this present disclosure is applicable. In other words, any aspect of the present disclosure can be defined by any combination of the described embodiments.

Thus, a first aspect of the present disclosure provides a medical watch for measuring physiological parameters of a subject. The medical watch comprising a top part having a top face that comprises a display. A central axis is defined normal to the top face. The medical watch further comprises a bottom part having a bottom face for facing the skin of a subject when the watch is worn and a peripheral member that is defined between the top face and the bottom face or used for coupling the bottom part and the top part of the medical watch. The peripheral member comprises or accommodates a pulse oximetry sensing surface and a first ECG sensing surface. Each of the sensing surfaces faces different radial directions with respect to the central axis. Namely, each of the directions that the sensing surfaces are facing is normal to the central axis and the direction of one of them is different than the other.

In some embodiments of the medical watch, the pulse oximetry sensing surfaces and the first ECG sensing surface are located at substantially two opposite portions of the peripheral member such they are facing substantially two opposite radial directions, therefore allowing to place simultaneously two fingers on the two sensors, typically the thumb and either the index or the middle finger, for performing simultaneous measurement from the two sensors.

It is to be noted that the term "substantially" in the context of opposite directions/positions of the sensing surfaces throughout the application should be understood as ±20° deviation from opposite portions of the peripheral member or ±20° deviation of radial directions that the sensing surfaces face with respect to the central axis. In some embodiments the term "substantially" should be understood as ±20° deviation from opposite portions of the inscribed circle of the shape of peripheral member or of the circumscribed circle of the shape of the peripheral member.

In some embodiments of the medical watch, the peripheral member further comprises a temperature sensing surface for measuring the temperature of the subject.

In some embodiments of the medical watch, the bottom face comprises a second ECG sensing surface for allowing ECG measurement by the first and second ECG sensing surfaces.

In some embodiments of the medical watch, the bottom face comprises a PPG sensing surface for measuring physiological parameters from a skin portion of the wrist of the subject.

In some embodiments of the medical watch, the peripheral member has a generally ring shape and the sensing surfaces are formed on the outer sider of the ring.

In some embodiments, the medical watch further comprising a connector for coupling to a continuous ECG unit. The connector is formed in the first ECG sensing element.

In some embodiments of the medical watch, the coupling is by means of a plug formed at a proximal end of flexible connecting element that comprises one or more active leads and a ground lead that is coupled, through the plug, to a ground connection in the medical watch.

In some embodiments, the medical watch further comprising a continuous ECG unit that comprises an extended ECG electrode electronically connected by a connecting element to the first ECG sensing surface. Namely, the extended ECG electrode is physically connected by connecting elements to the first ECG sensing surface. The extended ECG electrode is practically an ECG electrode that is connectable to the first ECG sensing surface and can be placed on any desired skin portion of the subject, therefore extending the options for locating the sensing portion related to the first ECG sensing surface. Thus, the ECG sensing capabilities of the watch are extended such that the extended ECG electrode can be attached constantly to a skin portion of a subject wearing the watch to perform continuous ECG measurement by the medical watch.

In some embodiments of the medical watch, the connecting element is further coupled to a ground connection formed in the medical watch.

In some embodiments of the medical watch, the peripheral member further comprises a sensing surface of a temperature sensor, the temperature sensor comprises said ground connection.

In some embodiments of the medical watch, at least part of the connecting element is a flexible PCB. In some embodiments, the flexible PCB portion of the connecting element is the portion that is tightly fitted over the peripheral member of the medical watch.

The continuous ECG unit comprises a casing made of or comprises an electrically insulating material. The casing is configured to (i) fit over the peripheral member and (ii) accommodate at least a part of the connecting element such that at least portions of the connecting element are disposed between the peripheral member and the casing. Thus, the casing maintains a constant contact of the connecting element with the first ECG sensing surface, and optionally also with the ground connection and prevents undesired contact of the user with the connecting element that may interrupt the ECG measurement.

In some embodiments of the medical watch, the casing is designed to allow accessibility to the pulse oximetry sensing surface. In a specific embodiment, the casing comprises an opening, an aperture or is shaped in a manner allowing to perform a measurement of the pulse oximetry sensor while being fitted over the peripheral member.

Yet another aspect of the present disclosure provides a continuous ECG unit couplable to a medical watch that comprises a first and second ECG sensing surfaces. The continuous ECG unit comprising an extended ECG electrode and a connecting element connected to the extended ECG electrode for allowing electronic connection with a first ECG sensing surface that is formed on an external portion of a watch, in particular that is formed on a peripheral member of watch.

In some embodiments of the continuous ECG unit the connecting element is further couplable to a ground connection formed in the medical watch.

In some embodiments of the continuous ECG unit, at least part of the connecting element is a flexible PCB.

In some embodiments, the continuous ECG unit further comprising a casing made of an electrically insulating material and is configured to (i) tightly fit over a peripheral member of the medical watch that comprises said first ECG sensing surface, namely fit over the peripheral rim of the medical watch such that it is secured in place; (ii) accommodate at least a part of the connecting element such that at least portions of the connecting element are disposed between the peripheral member and the casing, when being fitted over the peripheral member; and (iii) maintaining a bottom face of the medical watch, that comprises the second ECG sensing surface, exposed to allow constant contact between the second ECG sensing surface and the wrist of a subject wearing the watch with the continuous ECG unit.

In some embodiments, the portions of the connecting element that are disposed between the peripheral member and the casing are constituted by a flexible PCB.

In some embodiments of the continuous ECG unit, the casing is designed to allow access for additional one or more sensing elements of the medical watch.

In some embodiments of the continuous ECG unit, the casing comprises apertures allowing accessibility to said additional one or more sensing elements. The apertures are formed in the insulated material and are generally having the shape (may be a bit smaller or a bit larger) of the sensing elements they are allowing the access to.

In some embodiments of the continuous ECG unit, said additional one or more sensing elements comprises a pulse oximetry sensing surface and/or a temperature sensor sensing surface.

In some embodiments of the continuous ECG unit, the casing is designed to allow accessibility to the sensing surface of the pulse oximetry sensor.

In some embodiments of the continuous ECG unit, the casing comprises an opening, an aperture or is shaped in a manner allowing to perform a measurement of the pulse oximetry sensor while being fitted over the peripheral member.

Yet another aspect of the present disclosure provides a system for measuring physiological parameters of a subject. The system comprising a medical watch that comprises a top part having a top face that comprises a display. A central axis is defined normal to the top face. The medical watch further comprises a bottom part having a bottom face that faces the skin of a subject when the watch is worn, and a peripheral member defined between the top face and the bottom face or coupling the top part and the bottom part of the medical watch. A first ECG sensing surface is formed at the peripheral member and a second ECG sensing surface is formed at the bottom face. The system further comprises a continuous ECG unit of any of the above embodiments or any combination thereof, wherein the continuous ECG unit is electrically coupled to the first ECG sensing surface.

In some embodiments of the system, the connecting element is further coupled to a ground connection formed in the medical watch.

In some embodiments of the system, the peripheral member further comprises a sensing surface of a temperature sensor, and the temperature sensor comprises said ground connection.

Yet another aspect of the present disclosure provides a method for converting a medical watch to a continuous ECG measurement device. The medical watch comprises a first and second ECG sensing surfaces. The medical watch comprises a top face that comprises a display, a bottom face for contacting the skin of the subject and a peripheral member disposed therebetween. The peripheral member comprises or accommodates a first ECG sensing element, and the bottom face comprises a second ECG sensing element. The method comprises fitting a continuous ECG unit according to any one of the above-described embodiments on the peripheral member.

In some embodiments of the method, said fitting is such that the continuous ECG unit is fitted only on portions of the peripheral member.

### EMBODIMENTS

The following are optional embodiments and combinations thereof in accordance with aspects of the present disclosure:
By one embodiment, a medical watch for measuring physiological parameters of a subject is provided that comprises: a watch body defined between a top face that comprises a display, a bottom face for contacting the skin of the subject and a peripheral member disposed therebetween; wherein said peripheral member comprises or accommodates a pulse oximetry sensing element and a first ECG sensing element, each disposed on a different portion of said peripheral surface.

By one embodiment, the pulse oximetry sensing element and the first ECG sensing element are located at substantially two opposite portions of said peripheral member surface.

By one embodiment, said peripheral member further comprises a temperature sensing element for measuring the temperature of the subject.

By one embodiment, the bottom face comprises a second ECG sensing element for allowing ECG measurement between the first and second ECG sensing surfaces.

By one embodiment, the bottom face comprises a PPG sensing surface.

By one embodiment, the peripheral member is annular.

By one embodiment, the medical watch comprises a connector for coupling to a continuous ECG unit, being formed in the first ECG sensing element.

By one embodiment, the coupling is by means of a plug formed at a proximal end of flexible connecting element that comprises one or more active leads and a ground lead that is coupled, through the plug, to a ground connection in the medical watch.

By one embodiment, the peripheral member further comprises a sensing element of a temperature sensor, the temperature sensor comprises said ground connection.

By one embodiment, at least part of the connecting element is a flexible PCB.

By one embodiment, the continuous ECG unit comprises a casing made of an electrically non-conducting material and is configured to (i) fit over the peripheral member and (ii) accommodate at least a part of the connecting element such that at least portions of it are disposed between the peripheral member and the casing.

By one embodiment, the casing is designed to allow contact with the pulse oximetry sensing element.

By one embodiment, the continuous ECG unit is couplable to a medical watch that comprises a first and second ECG sensing surfaces, the continuous ECG unit comprises: an extended ECG electrode electronically connectable by a connecting element to a first ECG sensing element of an ECG electrode that is formed on a peripheral member of a watch.

By one embodiment, the connecting element is further couplable to a ground connection formed in the medical watch.

By one embodiment, at least part of the connecting element is a flexible PCB.

By one embodiment, the continuous ECG unit further comprising a casing made of an electrically non-conducting material and is configured to (i) fit over a peripheral member of the medical watch that comprises said first ECG sensing surface and (ii) accommodate at least a part of the connecting element such that at least portions of the connecting element are disposed between the peripheral member and the casing, when being fitted over the peripheral member.

By one embodiment, the casing is designed to allow contact with the sensing element of the pulse oximetry sensor.

By one embodiment, the system for measuring physiological parameters of a subject, comprising: a medical watch that comprises a watch body defined between a top face that comprises a display, a bottom face for contacting the skin of the subject and a peripheral member disposed between the bottom and top faces; wherein said peripheral member comprises or accommodates a first ECG sensing element, and a second ECG sensing element is formed at the bottom face; and comprising a continuous ECG unit.

By one embodiment, the ECG unit is electrically coupled to the first ECG sensing element.

By one embodiment, the connecting element is further coupled to a ground connection formed in the medical watch.

By one embodiment, the peripheral member further comprises a sensing surface of a temperature sensor, the temperature sensor comprises said ground connection.

By one embodiment, a method for converting a medical watch to a continuous ECG measurement device is provided. The medical watch comprises a first and second ECG sensing surfaces, wherein the medical watch comprises a top face that comprises a display, a bottom face for contacting the skin of the subject and a peripheral member disposed therebetween, said peripheral member comprises or accommodates a first ECG sensing element, and the bottom face comprises a second ECG sensing element; wherein the method comprises fitting a continuous ECG unit as defined above on the peripheral member.

By one embodiment, said fitting is such that the continuous ECG unit is fitted only on portions of the peripheral member.

By one embodiment, the medical watch used is that of any of the above embodiments.

### BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:
**Figs. 1A-1D** are schematic illustrations of different perspective views of a non-limiting example of an embodiment of the medical watch according to an aspect of the present disclosure.
**Figs. 2A-2D** are schematic illustrations of different perspective views a non-limiting example of an embodiment of the continuous ECG unit according to an aspect of the present disclosure. **Figs. 2A-2B** show the continuous ECG unit alone and **Figs. 2C-****2D** show the continuous ECG unit coupled to a medical watch.

### DETAILED DESCRIPTION

The following figures are provided to exemplify embodiments and realization of the invention of the present disclosure.

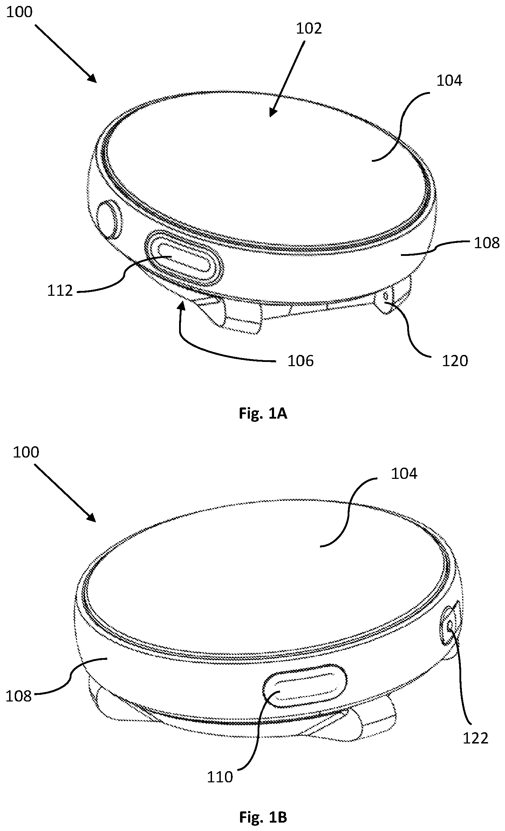

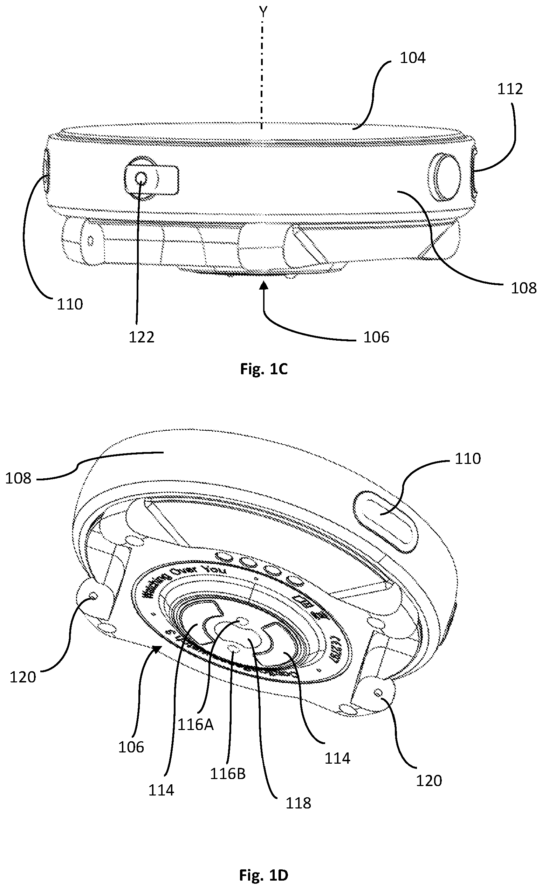

Reference is first being made to **Figs. 1A-1D****,** which are schematic illustrations of different perspective views of a non-limiting example of an embodiment of the medical watch according to an aspect of the present disclosure. The medical watch **100** is formed of a top face **102** that comprises a display **104** of the watch **100,** a bottom face **106** and a peripheral member **108** that is defined between the top face **102** and the bottom face **106.** A central axis **Y** is defined normal to the top face **102.** The peripheral member **108** extends over the entire periphery of the medical watch **100.** The peripheral member **108** comprises a first ECG sensing surface **110** of an ECG electrode and a pulse oximetry sensing surface **112** of a pulse oximetry sensor. The bottom face **106** comprises a second ECG sensing surface **114** that is designed to continuously contact the skin of the subject when the watch **100** is worn by the subject. Thus, by placing a finger on the first ECG sensing surface **110,** an ECG measurement can be taken. The watch **100** is designed to allow simultaneous measurement of ECG and pulse oximetry by placing simultaneously a first finger on the first ECG sensing surface **110** and a second finger on the pulse oximetry sensing surface **112.** To allow the simultaneous measurement, the first ECG sensing surface **110** and the pulse oximetry sensing surface **112** are formed at generally opposite positions of the peripheral member **108,** facing substantial opposite radial directions with respect to the central axis **Y.** In this example, the peripheral member **108** is circular and the first ECG sensing surface **110** and the pulse oximetry sensing surface **112** are formed at generally opposite arch sections defined by the peripheral member **108.**

The bottom face **106** further comprises a PPG sensor that includes two light sources **116A** and **116B** for illuminating the skin of the subject and a light detector **118** for receiving light from the skin in response of the illumination of the light sources. The light sources **116A** and **116B** can illuminate with the same wavelength range or illuminate with two different wavelength ranges. The bottom face **106** further comprises straps arrangements **120** for receiving straps to allow fastening the watch **100** over a wrist of a subject.

The peripheral member further comprises a temperature sensing surface **122** of a temperature sensor for allowing measuring of body temperature of the subject.

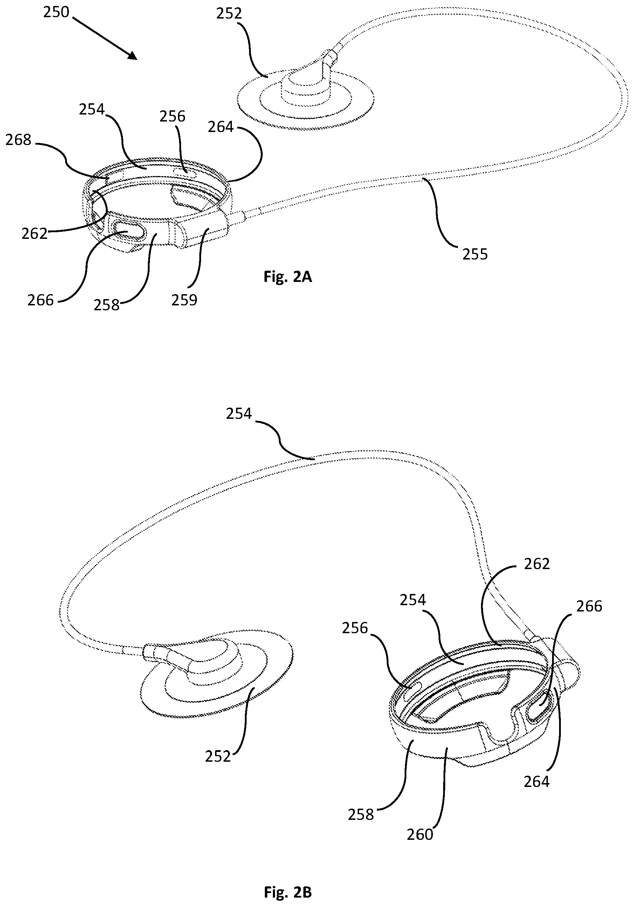

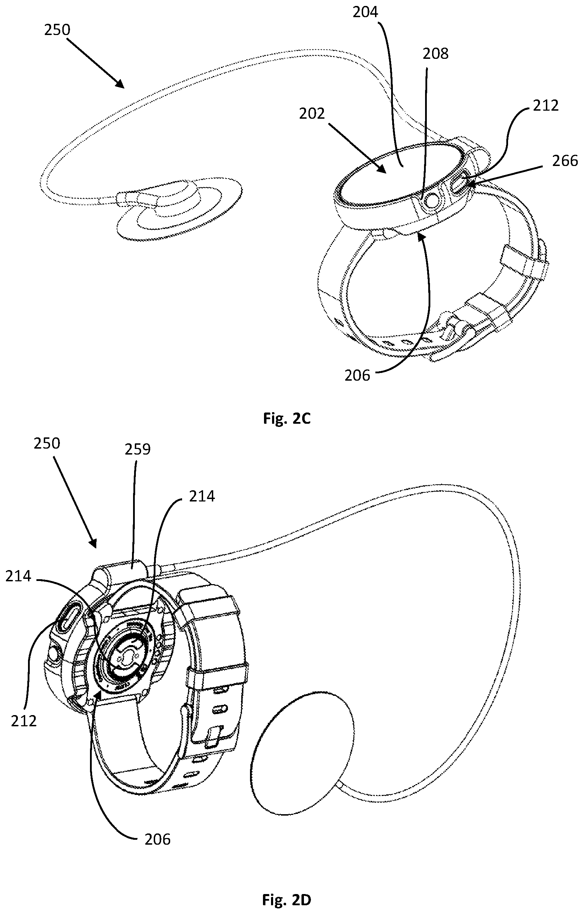

Reference is now being made to **Figs. 2A-2B****,** which are schematic illustrations of different perspective views a non-limiting example of an embodiment of the continuous ECG unit according to an aspect of the present disclosure. The continuous ECG unit **250** comprises an extended ECG electrode **252** that is placeable on a desired skin portion of a subject. The extended ECG electrode **252** is electronically coupled to a connecting element **254** that includes a first electronic pad **256** configured for electronically coupling with an ECG sensing surface of a watch. By electronically coupling the extended ECG electrode **252** to an ECG sensing surface of a watch, the ECG sensing surface of the watch becomes the extended ECG sensing surface. By doing that, the extended ECG electrode can be attached to a skin portion of the subject to provide a continuous ECG measurement by the watch, namely rendering the watch to a Holter device.

A part of the connecting element **254** is accommodated in an insulating casing **258** that is designed to tightly fit over the watch and is made of an electrically insulating material. Another part of the connecting element **254** is a flexible cord **255** that couples the extended ECG electrode **252** and the insulating casing **258.** The insulating casing **258** comprises peripheral wall **260** having an inner side **262** to which a part of the connecting element **254** is attached to and external side **264.** Thus, the insulating casing **258** insulates the connecting element **254** and prevents undesired contact between the subject and the connecting element that may interfere with the measurement. The insulating casing **258** is designed with an aperture **266** for allowing a subject to contact a sensing surface of the watch therethrough for performing a measurement, for example a pulse oximetry measurement.

The insulating casing **258** comprises a port **259** that is configured to receive the end of the flexible cord **255** to allow transition of the connecting element into the inner side **262** of the insulating casing **258.**

The connecting element **254** further comprises a second electronic pad **268** for contacting a ground connection that is formed in the watch. It is to be noted that the connecting element **254** is at least partially formed of a flexible PCB.

The insulating casing **258** is designed to fit in a specific manner over a peripheral member of a watch to ensure the alignment of the electronic pads **256** and **268** with the first ECG sensing surface and the ground connection, respectively.

Reference is now being made to **Figs. 2C-2D****,** which show the continuous ECG unit **250** being fitted over a peripheral member of a watch. The peripheral member **208** is defined between a top face **202** that is formed with a display **204** and a bottom face **206** that includes a second ECG sensing surface **214** of a second ECG electrode. The peripheral member **208** comprises the first ECG sensing surface and the ground connection to which the electronic pads of the continuous ECG unit is connected to. The peripheral member **208** further comprises a pulse oximetry sensing surface **212** that is accessible via the aperture **266** in the insulating casing **258.**

## Claims

1. A unit (250) couplable to a medical watch that comprises a first and second ECG sensing surfaces for allowing continuous ECG measurement by the medical watch, the continuous ECG unit comprising:
an extended ECG electrode (252) electronically connectable by a connecting element (254) to said first ECG sensing surface of an ECG electrode that is formed on a peripheral member of a watch;
wherein the unit is **characterized by**:
a casing (258) made of an insulating material and is configured to (i) tightly fit over a peripheral member of the medical watch that comprises said first ECG sensing surface, (ii) accommodate at least a part of the connecting element (254) such that at least portions of the connecting element (254) are disposed between the peripheral member and the casing (258), when being fitted over the peripheral member so as to maintain a constant contact of the connecting element with the first ECG sensing surface, and (iii) maintaining a bottom face of the medical watch that comprises the second ECG surface exposed.

2. The unit of claim 1, wherein the connecting element (254) is further couplable to a ground connection formed in the medical watch.

3. The unit of claim 1 or 2, wherein at least part of the connecting element (254) is a flexible PCB.

4. The unit of claim 1, wherein the casing (258) is designed to allow access for additional one or more sensing elements of the medical watch.

5. The unit of claim 4, wherein the casing (258) comprises apertures (266) allowing accessibility to said additional one or more sensing elements.

6. The unit of claim 4 or 5, wherein said additional one or more sensing elements comprises a pulse oximetry sensing surface and/or a temperature sensor sensing surface.

7. The unit of claim 6, wherein said at least a part of the connecting element is constituted by a first electronic pad (256) configured for electronically coupling with an ECG sensing surface of a watch.

8. A system for measuring physiological parameters of a subject, comprising:
a medical watch that comprises
a watch body defined between a top face (202) that comprises a display, a bottom face (206) for contacting the skin of the subject and a peripheral member (208) disposed between the bottom and top faces;
wherein said peripheral member (208) comprises or accommodates a first ECG sensing surface, and a second ECG sensing surface is formed at the bottom face; and
a unit (250) of any one of claims 1-7.

9. The system of claim 8, wherein the unit (250) is electrically coupled to the first ECG sensing element.

10. The system of claim 8 or 9, wherein the connecting element (254) is further coupled to a ground connection formed in the medical watch.

11. The system of claim 10, wherein the peripheral member (208) further comprises a sensing surface of a temperature sensor, the temperature sensor comprises said ground connection.

12. A method for converting a medical watch to a continuous ECG measurement device, the medical watch comprises
a first and second ECG sensing surfaces, wherein the medical watch comprises a top face (202) that comprises a display, a bottom face (204) for contacting the skin of the subject and a peripheral member (208) disposed therebetween;
wherein said peripheral member (208) comprises or accommodates a first ECG sensing surface, and the bottom face comprises a second ECG sensing surface;
wherein the method comprises fitting a unit (250) according to any one of claims 1-7 on the peripheral member (208).

13. The method of claim 12, wherein said fitting is such that the unit (250) is fitted only on portions of the peripheral member (208).

## Patentansprüche

1. Eine Einheit (250), die mit einer medizinischen Uhr koppelbar ist und eine erste und eine zweite EKG-Sensorfläche umfasst, um eine kontinuierliche EKG-Messung durch die medizinische Uhr zu ermöglichen. Die Einheit für kontinuierliches EKG umfasst:
eine ausgedehnte EKG-Elektrode (252), die über ein Verbindungselement (254) elektronisch mit der genannten ersten EKG-Sensorfläche an einer EKG-Elektrode verbindbar ist, Elektrode, die auf einem Peripherieelement einer Uhr ausgebildet ist;
wobei die Einheit **gekennzeichnet ist durch**:
ein Gehäuse (258), das aus einem isolierenden Material besteht und so konfiguriert ist, dass es (i) fest über ein Peripherieelement der medizinischen Uhr passt, das die genannte erste EKG-Sensorfläche umfasst, (ii) mindestens einen Teil des Verbindungselements (254) aufnimmt, sodass zumindest Teile des Verbindungselements (254) zwischen dem Peripherieelement und dem Gehäuse (258) angeordnet sind, wenn es über das Peripherieelement geschoben wird, um einen konstanten Kontakt des Verbindungselements mit der ersten EKG-Sensorfläche aufrechtzuerhalten, und (iii) Beibehaltung einer Unterseite der medizinischen Uhr, die die zweite EKG-Fläche aufweist, freilegt.

2. Die Einheit gemäß Anspruch 1, wobei das Verbindungselement (254) zusätzlich mit einem in der medizinischen Uhr ausgebildeten Masseanschluss koppelbar ist.

3. Die Einheit gemäß Anspruch 1 oder 2, wobei zumindest ein Teil des Verbindungselements (254) eine flexible Leiterplatte (PCB) ist.

4. Die Einheit gemäß Anspruch 1, wobei das Gehäuse (258) so ausgestaltet ist, dass es den Zugang zu einem oder mehreren zusätzlichen Sensorelementen der medizinischen Uhr ermöglicht.

5. Die Einheit gemäß Anspruch 4, wobei das Gehäuse (258) Öffnungen (266) aufweist, die den Zugänglichkeit zu dem genannten oder zu mehreren zusätzlichen Sensorelementen ermöglichen.

6. Die Einheit gemäß Anspruch 4 oder 5, wobei die genannten zusätzlichen Sensorelemente eine Pulsoximetrie-Sensorfläche und/oder eine Temperatursensor-Sensorfläche umfassen.

7. Die Einheit gemäß Anspruch 6, wobei genanntes zumindest ein Teil des Verbindungselements aus einem ersten elektronischen Pad (256) besteht, das für die elektronische Kopplung mit einer EKG-Sensorfläche einer Uhr konfiguriert ist.

8. Ein System zur Messung physiologischer Parameter eines Subjekts, umfassend:
eine medizinische Uhr, umfassend
ein Uhrengehäuse, das zwischen einer Oberseite (202), die ein Display umfasst, einer Unterseite (206) zum Kontakt mit der Haut des Subjekts und einem peripheren Element (208), das zwischen der Unterseite und der Oberseite angeordnet ist, definiert ist;
wobei das genannte Peripherieelement (208) eine erste EKG-Sensorfläche aufweist oder beherbergt und eine zweite EKG-Sensorfläche an der Unterseite ausgebildet ist; und
eine Einheit (250) gemäß einem der Ansprüche 1-7.

9. Das System gemäß Anspruch 8, wobei die Einheit (250) elektrisch mit dem ersten EKG-Sensorelement gekoppelt ist.

10. Das System gemäß Anspruch 8 oder 9, wobei das Verbindungselement (254) zusätzlich mit einem in der medizinischen Uhr ausgebildeten Masseanschluss gekoppelt ist.

11. Das System gemäß Anspruch 10, wobei das Peripherieelement (208) zusätzlich eine Sensorfläche eines Temperatursensors aufweist, wobei der Temperatursensor den genannten Masseanschluss umfasst.

12. Ein Verfahren zum Umbau einer medizinischen Uhr in ein Gerät zur kontinuierlichen EKG-Messung. Die medizinische Uhr umfasst
eine erste und eine zweite EKG-Sensorfläche, wobei die medizinische Uhr eine Oberseite (202) mit Display, eine Unterseite (204) zum Kontakt mit der Haut des Subjekts und ein dazwischen angeordnetes Peripherieelement (208) umfasst;
wobei das genannte Peripherieelement (208) eine erste EKG-Sensorfläche umfasst, und die Unterseite eine zweite EKG-Sensorfläche umfasst;
wobei das Verfahren die Befestigung einer Einheit (250) gemäß einem der Ansprüche 1 bis 7 am Peripherieelement (208) umfasst.

13. Das Verfahren gemäß Anspruch 12, wobei die genannte Befestigung so erfolgt, dass die Einheit (250) nur auf Teile des Peripherieelements (208) aufgesetzt wird.

## Revendications

1. Unité (250) pouvant être couplée à une montre médicale et comprenant une première et une seconde surfaces de détection d'ECG permettant une mesure continue de l'ECG par la montre médicale, l'unité d'ECG continue comprenant :
une électrode ECG étendue (252) connectable électroniquement par un élément de connexion (254) à ladite première surface de détection ECG d'une électrode ECG qui est formée sur un élément périphérique d'une montre ;
dans laquelle l'unité est **caractérisée par** :
un boîtier (258) en matériau isolant et configuré pour (i) s'ajuster étroitement sur un élément périphérique de la montre médicale qui comprend ladite première surface de détection ECG, (ii) loger au moins une partie de l'élément de connexion (254) de telle sorte qu'au moins des parties de l'élément de connexion (254) sont disposées entre l'élément périphérique et le boîtier (258), lorsqu'il est ajusté sur l'élément périphérique de manière à maintenir un contact constant de l'élément de connexion avec la première surface de détection ECG, et (iii) maintenir une face inférieure de la montre médicale qui comprend la seconde surface ECG exposée.

2. Unité selon la revendication 1, dans laquelle l'élément de connexion (254) peut en outre être couplé à une connexion à la terre formée dans la montre médicale.

3. Unité selon la revendication 1 ou 2, dans laquelle au moins une partie de l'élément de connexion (254) est un circuit imprimé flexible.

4. Unité selon la revendication 1, dans laquelle le boîtier (258) est conçu pour permettre l'accès à un ou plusieurs éléments de détection supplémentaires de la montre médicale.

5. Unité selon la revendication 4, dans laquelle le boîtier (258) comprend des ouvertures (266) permettant l'accès au(x)dit(s) élément(s) de détection supplémentaire(s).

6. Unité selon la revendication 4 ou 5, dans laquelle ledit ou lesdits éléments de détection supplémentaires comprennent une surface de détection d'un oxymètre de pouls et/ou une surface de détection d'un capteur de température.

7. Unité selon la revendication 6, dans laquelle au moins une partie de l'élément de connexion est constituée d'un premier plot électronique (256) configuré pour se coupler électroniquement à une surface de détection d'ECG d'une montre.

8. Système de mesure des paramètres physiologiques d'un sujet, comprenant :
une montre médicale qui comprend
un corps de montre défini entre une face supérieure (202) qui comprend un affichage, une face inférieure (206) destinée à entrer en contact avec la peau du sujet et un élément périphérique (208) disposé entre les faces inférieure et supérieure ;
dans lequel ledit élément périphérique (208) comprend ou loge une première surface de détection ECG, et une seconde surface de détection ECG est formée sur la face inférieure ; et
une unité (250) selon l'une quelconque des revendications 1 à 7.

9. Système selon la revendication 8, dans lequel l'unité (250) est couplée électriquement au premier élément de détection d'ECG.

10. Système selon la revendication 8 ou 9, dans lequel l'élément de connexion (254) est en outre couplé à une connexion à la terre formée dans la montre médicale.

11. Système selon la revendication 10, dans lequel l'élément périphérique (208) comprend en outre une surface de détection d'un capteur de température, ledit capteur de température comprenant ladite connexion à la terre.

12. Procédé de conversion d'une montre médicale en un dispositif de mesure d'ECG en continu, la montre médicale comprenant
une première et une seconde surfaces de détection d'ECG, dans laquelle la montre médicale comprend une face supérieure (202) qui comprend un affichage, une face inférieure (204) destinée à entrer en contact avec la peau du sujet et un élément périphérique (208) disposé entre celles-ci ;
dans lequel ledit élément périphérique (208) comprend ou loge une première surface de détection ECG, et la face inférieure comprend une seconde surface de détection ECG ;
dans lequel le procédé comprend le montage d'une unité (250) selon l'une quelconque des revendications 1 à 7 sur l'élément périphérique (208).

13. Procédé selon la revendication 12, dans lequel ledit montage est tel que l'unité (250) est montée uniquement sur des parties de l'élément périphérique (208).
